# EUROPÄISCHE PATENTANMELDUNG

(11) **EP 2 777 798 A1**
(43) Veröffentlichungstag der Anmeldung: **17.09.2014**
(21) Anmeldenummer: 14000526.5
(22) Anmeldetag: 13.02.2014
(51) Int. Cl.: B01D 53/22, C10L 3/10

(54) **Verfahren zur Gewinnung von hochreinem Methan aus Biogas sowie Anlage zur Durchführung dieses Verfahrens**

(30) Priorität: 11.03.2013 DE 102013004079
(71) Anmelder: Eisenmann AG, 71032 Böblingen (DE)
(72) Erfinder: Graf, Lukas, 70193 Stuttgart (DE)
(74) Vertreter: Ostertag, Ulrich

(57) **Zusammenfassung**

Es werden ein Verfahren und eine Anlage (1) zur Gewinnung von hochreinem Methan aus Biogas beschrieben. Das aus einem Fermenter kommende Biogas wird nach Verdichtung mindestens einer eine selektivpermeable Membran aufweisenden Membraneinheit (4, 5, 6) zugeführt, der in bekannter Weise ein einen erhöhten Methan aufweisender Gasstrom und ein einen verringerten Methananteil enthaltender Gasstrom entnommen werden können. Um dauerhaft eine gleichbleibende Qualität des erhaltenen Methans sicherzustellen, wird in der Auslassleitung (3), welche das hochreine Produktgas abführt, ein Qualitätssensor (17) angeordnet. Eine in ihrer Leistung regelbare Vakuumpumpe (15) ist mit der Unterdruckseite einer der Membraneinheiten (5) verbunden. Sie wird von einer Steuereinheit (26) angesteuert, die ihr Eingangssignal von dem Qualitätssensor (17) in der Auslassleitung (3) erhält. Wenn der von dem Qualitätssensor (17) gemessene Methananteil unter einen gewissen Wert abfällt, wird die Leistung der Vakuumpumpe (15) hochgeregelt, so dass die Druckdifferenz an der Membran der Membraneinheit (5) ansteigt, was die Selektivität des Brennprozesses verbessert. Auf diese Weise wird die Qualität des Produktgases ständig aufrecht erhalten.

## Beschreibung

Die Erfindung betrifft ein Verfahren zur Gewinnung von hochreinem Methan aus Biogas, bei dem
a) das aus einem Fermenter kommende Biogas verdichtet wird;
b) das verdichtete Biogas mittels mindestens einer selektiv permeablen Membran in mindestens zwei Gasströme aufgeteilt wird, von denen einer einen erhöhten Methananteil und der andere einen verringerten Methananteil enthält;
   wobei
c) an der Membran eine Druckdifferenz erzeugt wird;
d) ein einen ausreichenden Methananteil enthaltender Gasstrom als Produktgas einer weiteren Verwendung zugeführt wird;
sowie
eine Anlage zur Gewinnung von hochreinem Methan aus Biogas mit
a) einem Verdichter, in dem das aus einem Fermenter kommende Biogas verdichtet wird;
b) mindestens einer Membraneinheit, die aufweist:
   ba) einen Einlass für das zu trennende Gasgemisch;
   bb) eine selektiv permeable Membran, deren eine Seite im Betrieb einem höheren Druck und deren andere Seite einem niedrigeren Druck ausgesetzt ist;
   bc) einem Retentatauslass auf der Hochdruckseite der Membraneinheit, über den ein Gasstrom entnommen werden kann, der einen erhöhten Anteil an Methan enthält;
   bd) einen Permeatauslass auf der Niedrigdruckseite der Membraneinheit, über den ein Gasstrom entnommen werden kann, der einen erniedrigten Anteil an Methan enthält;
c) einer ersten Auslassleitung, über welche ein aus hochreinem Methan bestehender Produktgasstrom einer weiteren Verwendung zuführbar ist;
d) einer zweiten Auslassleitung, über welche ein nur noch einen geringen Methananteil aufweisender Offgasstrom an die Umgebung abgegeben werden kann.

Ein derartiges Verfahren sowie eine derartige Anlage sind aus der WO 2012/000727 A1 bekannt. In dieser Druckschrift werden vor allem verschiedene Verschaltungen mehrerer Membraneinheiten beschrieben, mit denen ein Produktgas erzielt werden kann, das einen sehr hohen Anteil an Methan enthält. Gleichzeitig soll der Offgasstrom nur noch möglichst wenig Methan enthalten. In dieser Druckschrift ist zwar erwähnt, dass die Druckdifferenz an der selektiv permeablen Membran deren Selektivität beeinflusst. Von dieser Erkenntnis wird dann aber weiter kein Gebrauch gemacht. Vielmehr wird als Weg, die Selektivität des Prozesses zu erhöhen, eine bestimmte Art der Rückführung von Gasströmen gewählt. Eine dauerhafte Überwachung der Qualität des Produktgases und insbesondere eine Regelung auf einen bestimmten Methananteil in diesem findet hier nicht statt.

In der EP 1 324 815 B1 ist am Beispiel einer Anlage, die Stickstoff mit hoher Reinheit erzeugt, beschrieben, dass die Qualität des Produktgases durch einen Sensor laufend überwacht werden kann, wobei dann die Druckdifferenz an mindestens einer Membraneinheit zur Gewährleistung einer bestimmten Produktgasqualität entsprechend verändert wird. Diese Veränderung geschieht in der EP 1 324 815 B1 durch Änderung der Drehzahl des Kompressors, der das zugeführte Gasgemisch verdichtet, also auf der Hochdruckseite der Membran, was zur Folge hat, dass sich bei einem Nachregelprozess der die Anlage durchsetzende Volumenstrom verändert. Dies ist im Allgemeinen weniger erwünscht.

Aufgabe der vorliegenden Erfindung ist es, ein Verfahren und eine Anlage der eingangs genannten Art so auszugestalten, dass während des Betriebes dauerhaft eine bestimmte Qualität des Produktgases aufrecht erhalten werden kann, ohne dass beim Regelprozess nennenswerte Volumenstromänderungen eintreten würden.

Diese Aufgabe wird, was das Verfahren angeht, dadurch gelöst, dass
e) der Methangasanteil im Produktgas laufend gemessen wird;
f) beim Unterschreiten eines vorgegebenen Wertes des Menthananteiles im Produktgas die Druckdifferenz an
einer Membran dadurch erhöht wird, dass auf der Niedrigdruckseite der Membran ein Vakuum erzeugt oder ein bestehendes Vakuum erhöht wird.

Die Erfindung geht also gedanklich eine Strecke des Weges mit, der in der EP 1 324 815 B1 für andere Gase als Biogase vorgezeichnet ist: Sie sieht einen Sensor vor, der die Qualität des erzeugten Produktgases überwacht. Die zur Veränderung der Selektivität der selektiv permeablen Membran erforderliche Veränderung der an dieser anliegenden Druckdifferenz wird nunmehr aber nicht auf der Hochdruckseite der Membran sondern auf der Niedrigdruckseite bewirkt. Dies hat zur Folge, dass bei Nachregelprozessen keine nennenswerten Veränderungen des Volumenstromes eintreten. Überraschend ist die erfindungsgemäße Vorgehensweise unter anderem deshalb, weil auf der Niedrigdruckseite verständlicherweise sehr viel weniger Veränderungen des Druckes als auf der Hochdruckseite möglich sind. Erfindungsgemäß wurde erkannt, dass die möglichen Druckveränderungen für den gewünschten Regelprozess bei der Herstellung von hochreinem Methan ausreichen.

Die oben genannte Aufgabe wird, was die Anlage angeht dadurch gelöst, dass
e) ein erster Qualitätssensor in der ersten Auslassleitung vorgesehen ist, der ein für den Methananteil im Produktgas repräsentatives Signal erzeugt;
f) eine Vakuumpumpe vorgesehen ist, die saugseitig mit der Niedrigdruckseite einer Membraneinheit und druckseitig direkt oder indirekt mit dem Einlass des Verdichters verbunden ist;
g) eine erste Steuereinrichtung vorgesehen ist, welcher das Signal des ersten Qualitätssensors zuführbar ist und deren Ausgangssignal die Saugleistung der Vakuumpumpe so regelt, dass ein vorgegebener Wert für den Menthananteil in dem Produktgas nicht unterschritten wird.

Die Vorteile der erfindungsgemäßen Anlage entsprechen sinngemäß den oben genannten Vorteilen des erfindungsgemäßen Verfahrens.

Bei einer bevorzugten Ausführungsform der Erfindung sind mindestens zwei Membraneinheiten vorgesehen,
wobei
der Einlass der ersten Membraneinheit mit dem Auslass des Verdichters verbunden ist;
der Retentatauslass der ersten Membraneinheit mit dem Einlass der zweiten Membraneinheit verbunden ist;
die Vakuumpumpe mit dem Permeatauslass der zweiten Membraneinheit verbunden ist;
der Retentatauslass der zweiten Membraneinheit mit der ersten Auslassleitung für das Produktgas verbunden ist.

Die Verschaltung zweier in Serie liegender Membraneinheiten ist zwar aus der oben erwähnten WO 2012/000727 A1 bekannt. Sie dient der Erzeugung eines stärker mit Methan angereicherten Produktgases. Im vorliegenden Zusammenhang interessant ist jedoch die erfindungsgemäße Beeinflussung nicht der ersten sondern der zweiten Membraneinheit durch Erzeugung eines Unterdrucks an der Niedrigdruckseite.

Besonders bevorzugt schließlich ist eine Anlage mit zwei Membraneinheiten, bei der der Permeatauslass der ersten Membraneinheit mit dem Einlass einer dritten Membraneinheit verbunden ist, deren Retentatauslass über ein steuerbares Regelventil direkt oder indirekt mit dem Einlass des Verdichters und deren Permeatauslass mit der zweiten Auslassleitung für das Offgas verbunden ist;
wobei
in der zweiten Auslassleitung für das Offgas ein zweiter Qualitätssensor vorgesehen ist, der ein für den Methananteil im Offgas repräsentatives Signal erzeugt;
eine zweite Steuereinheit vorgesehen ist, welcher das Signal des zweiten Qualitätssensors zuführbar ist und deren Ausgangssignal das Regelventil so ansteuert, dass ein vorgegebener Wert für den Methananteil im Offgas nicht überschritten wird.

Die Verschaltung zweier Membraneinheiten in der Weise, dass der Permeatanschluss der ersten Membraneinheit noch einmal dem Einlass einer weiteren Membraneinheit zugeführt wird, ist ebenfalls in der WO 2012/000727 A1 beschrieben. Sie dient der Gewinnung von Methan, das im Permeat der ersten Membraneinheit noch enthalten ist und sonst verloren ging. Erfindungsgemäß wird anders als in dieser Druckschrift die Produktqualität des Offgases überwacht, wobei es hier darauf ankommt, dass der Methananteil nicht zu hoch ansteigt. Die Regelung der Selektivität der dritten Membraneinheit geschieht nunmehr aber zwangsläufig druckseitig, aber nicht über eine Veränderung der Verdichterdrehzahl sondern über ein Regelventil, das umso weiter geöffnet wird, umso kleiner der Druck auf der Hochdruckseite der entsprechenden Membran sein soll.

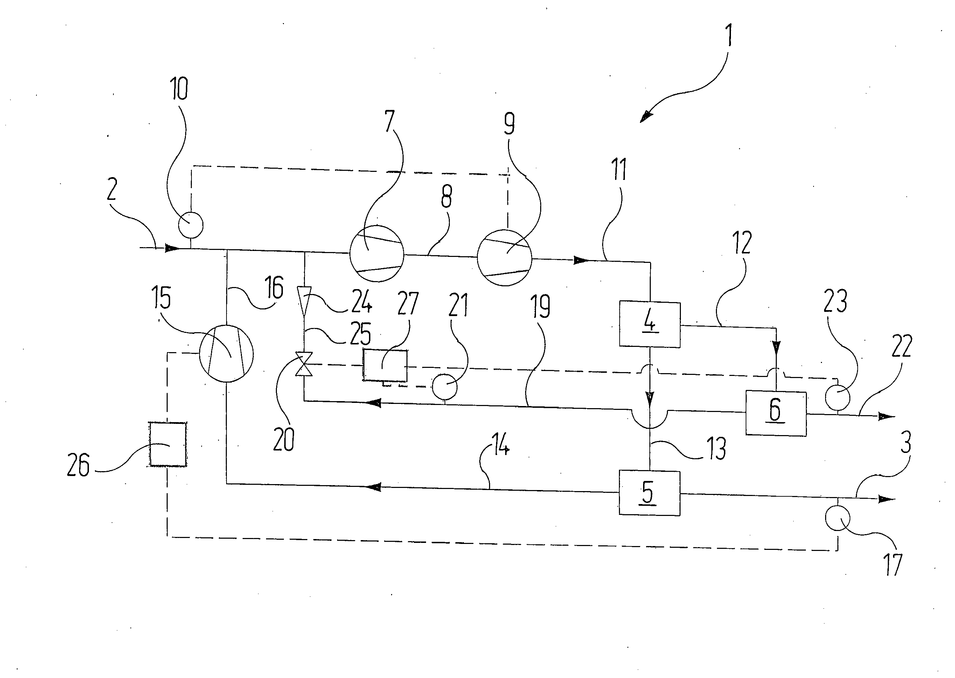

Ein Ausführungsbeispiel der Erfindung wird nachfolgend anhand der Zeichnung näher erläutert; die einzige Figur zeigt schematisch das Layout einer Anlage zur Gewinnung von hochreinem Methan aus Biogas.

Die in der Zeichnung dargestellte und insgesamt mit dem Bezugszeichen 1 versehene Anlage zur Gewinnung von hochreinem Methan erhält über eine Leitung 2 von einem nicht dargestellten Fermenter Biogas, also ein Mischgas, das zu wesentlichen Teilen aus Stickstoff, Sauerstoff, CO₂, CO und eben Methan besteht. In der Anlage 1 werden auf eine Weise, die weiter unten näher beschrieben wird, im Wesentlichen alle Bestandteile des Biogases mit Ausnahme von Methan abgetrennt, das in hochreiner Form über eine erste Auslassleitung 3 abgegeben wird. Die Qualität des hier abgegebenen Methans liegt bei über 96%, vorzugsweise über 98%, so dass es bei Bedarf direkt in ein Erdgasversorgungssystem eingegeben werden kann. Geringfügige verbleibende Bestandteile anderer Gase können 2% CO₂ und/oder 2% N₂ sein, Restbestandteile, die der Verwertung des gereinigten Gases in einem Erdgassystem nicht entgegenstehen.

Die Reinigung des über die Leitung 2 zugeführten Biogases geschieht mit Hilfe von drei Membraneinheiten 4, 5 und 6, die in bestimmter, unten beschriebener Weise miteinander verschaltet sind. Der Aufbau dieser Membraneinheiten 4, 5, 6 ist konventionell, braucht also nicht näher beschrieben zu werden. Hier genügt es zu wissen, dass jede dieser Membraneinheiten 4, 5, 6 eine selektiv permeable Membran aufweist, welche einen ersten Raum, welchem das zu trennende Gas über einen Einlass zugeführt und dem das schwerer permeierende Gas (Retentat) über einen Retentatauslass entnommen wird, von einem Raum trennt, in welchen das besser permeierende Gas (Permeat) durch die Membran hindurchtritt und aus dem es über einen Permetatauslasss entnommen wird.

Die Retentatseiten der Membraneinheiten 4, 5, 6 befinden sich dabei auf höherem Druck als die Permeatseite; diese Druckdifferenz stellt die "treibende Kraft" des Trennvorganges dar. Bekannt ist, dass die Selektivität des Trennvorganges mit der an der Membran liegenden Druckdifferenz zunimmt.

Nach diesen Vorbemerkungen sei nun die detaillierte Verschaltung der verschiedenen Membraneinheiten 4, 5, 6 erläutert.

Das über die Leitung 2 zugeführte Biogas wird zunächst einem Gebläse 7 zugeführt, das auslassseitig über eine Leitung 8 mit einem Verdichter 9 verbunden ist. Ein Drucksensor 10 misst den Druck des Biogases in der Leitung 2 und steuert die Drehzahl des Verdichters 9, also dessen Förderleistung, proportional.

Das vom Verdichter 9 verdichtete Biogas wird nunmehr über den Einlass der ersten Membraneinheit 4 zugeführt; dies geschieht über eine Leitung 11. Der Permeatauslass der ersten Membraneinheit 4 ist über eine weitere Leitung 12 mit dem Einlass der dritten Membraneinheit 6 verbunden. Das Retentat der ersten Membraneinheit 4 dagegen wird über eine Leitung 13 dem Einlass der zweiten Membraneinheit 5 zugeführt. Diese Membraneinheit 5 ist außerdem permeatseitig über eine Leitung 14 mit der Saugseite einer Vakuumpumpe 15 verbunden, welche das angesaugte Permeat der zweiten Membraneinheit 5 über eine Leitung 16 wieder der Leitung 2 zuführt und somit erneut gemeinsam mit dem über die Leitung 2 zuströmenden Biogas vom Gebläse 7 in der Zeichnung nach rechts zum Verdichter 9 gefördert wird.

Der Retentatauslass der zweiten Membraneinheit 5 ist mit der ersten Auslassleitung 3 verbunden, mit der ein Sensor 17 kommuniziert, der die Qualität des ausströmenden, gereinigten Produktgases misst. Es handelt sich dabei vorzugsweise um einen optischen Sensor, der auf Methan empfindlich ist. Derartige Sensoren sind bekannt und im Handel erhältlich; sie brauchen daher nicht näher erläutert werden.

Das Ausgangssignal des Sensors 17 wird einer ersten Steuerungseinrichtung 26 zugeführt, welche ein Ausgangssignal abgibt, das die Saugleistung der Vakuumpumpe 15 bestimmt. In der Leitung 14, welche den Permeatauslass der zweiten Membraneinheit 5 mit der Vakuumpumpe 15 verbindet, kann sich zudem ein nicht dargestellter Drucksensor befinden, dessen Ausgangssignal ebenfalls der ersten Steuerungseinrichtung 26 zugeleitet wird und dessen Funktion später deutlich wird.

Es bleibt, die restliche Verschaltung der dritten Membraneinheit 6 zu erläutern:
Das Retentat der dritten Membraneinheit 6 wird über eine Leitung 19 einem Regelventil 20 zugeführt. Ein Drucksensor 21 überwacht den Druck der Retentatströmung in der Leitung 19.

Das Permeat der Membraneinheit 6 wird über eine zweite Auslassleitung 22 als Offgas abgeführt und verworfen. Die Qualität dieses Offgases wird von einem zweiten Qualitätssensor 23 kontrolliert, der ebenso wie der oben erwähnte erste Qualitätssensor 17 beschaffen sein kann. Insbesondere kann es sich also hier um einen optischen Sensor mit Empfindlichkeit für Methan handeln. Die Ausgangssignale des zweiten Qualitätssensors 23 und des Drucksensors 21 werden einer zweiten Steuerungseinrichtung 27 zugeleitet, die grundsätzlich mit der oben schon erwähnten ersten Steuerungseinrichtung 26 oder der übergeordneten Anlagensteuerung zusammengefasst sein kann und deren Ausgangssignal das Regelventil 20 in weiter unten beschriebenen Weise beaufschlagt.

Ausgangsseitig ist das Regelventil 20 über ein Entspannungsventil 24, das in einer Leitung 25 liegt, ebenfalls mit dem Eingang des Gebläses 7 verbunden.

Die Funktionsweise der oben beschriebenen, drei verschaltete Membraneinheiten 4, 5, 6 umfassenden Anlage 1 ist wie folgt:
Das dem Fermenter entnommene Biogas wird, wie schon erwähnt, über das Gebläse 7 angesaugt und vermischt sich dabei im stationären Betrieb der Anlage 1 mit Gas, welches über die Leitungen 16 und 25 aus der zweiten Membraneinheit 5 bzw. der dritten Membraneinheit 6 zurückgeführt wird. Die Leistung des Verdichters 7 wird dabei über den von dem Drucksensor 10 gemessenen Druck bestimmt.

Durch den Verdichter 9 komprimiertes Gasgemisch gelangt zum Einlass der ersten Membraneinheit 4; das aufgrund der Selektivität der verwendeten Membran mit Methan angereicherte Retentat wird dem Einlass der zweiten Membraneinheit 5 über die Leitung 13 zugeleitet, wo erneut eine selektive Trennung stattfindet: Das in der erforderlichen Weise hauptsächlich Methan enthaltende Retentat der zweiten Membranstufe 5 ist das gewünschte Produktgas, welches über die erste Auslassleitung 3 beispielsweise einem Erdgasversorgungssystem zugeführt wird.

Die Qualität dieses Gases wird durch den ersten Qualitätssensor 17 kontrolliert. Sinkt diese Qualität unter einen gewünschten Wert ab, so sorgt die erste Steuerungseinrichtung 26, welcher das Signal des Qualitätssensors 17 zugeleitet wird, dafür, dass die Saugleistung der Vakuumpumpe 15 erhöht wird. Dies hat zur Folge, dass der Unterdruck auf der Permeatseite der zweiten Membraneinheit 5 wächst, was wiederum zu einer besseren Selektivität der in der zweiten Membraneinheit 5 befindlichen Membran führt. Konsequenz ist eine bessere Qualität des Produktgases auf der ersten Auslassleitung 3, was von dem Sensor 17 registriert wird. Dieser gibt dann ein entsprechendes Signal an die erste Steuerungseinrichtung 26, welche die Saugleistung der Vakuumpumpe 15 auf dem zur Aufrechterhaltung der Qualität des Produktgases erforderlichen Wert hält.

Mit dem optional vorhandenen, nicht dargestellten Drucksensor hat es folgende Bewandnis:
Für den Unterdruck auf der Permeatseite der zweiten Membraneinheit 5, der durch diesen Drucksensor gemessen wird, kann ein bestimmter unterster Wert vorgegeben werden. Wird dieser unterschritten, lässt sich die Trennwirkung der zweiten Membraneinheit 5 nicht mehr weiter nennenswert durch weitere Absenkung des Vakuums mittels der Vakuumpumpe 15 steigern. In einem solchen Falle müssen dann zu der zweiten Membraneinheit 5 gegebenenfalls weitere Membraneinheiten parallelgeschaltet oder der Betrieb der Anlage 1 muss unterbrochen werden, um die Membranen der zweiten Membraneinheit 5 austauschen zu können.

Die dritte Membraneinheit 6 dient dazu, im Permeat der ersten Membraneinheit 4 noch befindliches Methan soweit wie möglich zu verwerten. Wenn der zweite Qualitätssensor 23 in der zweiten Auslassleitung 22 einen zu hohen Gehalt an Methan feststellt, der verloren ginge, wird mit Hilfe des Regelventils 20 die Druckdifferenz an der Membran der dritten Membraneinheit 6 erhöht, also deren Selektivität verbessert. Hierzu wird der Druck auf der Leitung 19, gemessen durch den Drucksensor 21, entsprechend reduziert.

Ergebnis der oben geschilderten Regelvorgänge ist, dass einerseits über die zweite Auslassleitung 22 nur sehr wenig Methan in die Außenatmosphäre verlorengeht und dass andererseits über die erste Auslasseitung 3 ein hochwertiges Produktgas gewonnen wird, welches soviel Methan enthält, dass es gleichwertig zu Erdgas weiterverwendet werden kann.

## Patentansprüche

1. Verfahren zur Gewinnung von hochreinem Methan aus Biogas, bei dem
a) das aus einem Fermenter kommende Biogas verdichtet wird;
b) das verdichtete Biogas mittels mindestens einer selektiven permeablen Membran in mindestens zwei Gasströme aufgeteilt wird, von denen einer einen erhöhten Methananteil und der andere einen verringerten Methananteil enthält;
wobei
c) an der Membran eine Druckdifferenz erzeugt wird;
d) ein einen ausreichenden Methananteil enthaltender Gasstrom als Produktgas einer weiteren Verwendung zugeführt wird;
**dadurch gekennzeichnet, dass**
e) der Methangasanteil im Produktgas laufend gemessen wird;
f) bei Unterschreiten eines vorgegebenen Wertes des Methananteiles im Produktgas die Druckdifferenz an einer Membran dadurch erhöht wird, dass auf der Niedrigdruckseite der Membran ein Vakuum erzeugt oder ein bestehendes Vakuum erhöht wird.

2. Anlage zur Gewinnung von hochreinem Methan aus Biogas mit
a) einem Verdichter (9), in dem das aus einem Fermenter kommende Biogas verdichtet wird;
b) mindestens einer Membraneinheit (4, 5, 6), die aufweist:
ba) einen Einlass für das zu trennende Gasgemisch;
bb) eine selektiv permeable Membran, deren eine Seite im Betrieb einem höheren Druck und deren andere Seite einem niedrigeren Druck ausgesetzt ist;
bc) einen Retentatauslass auf der Hochdruckseite der Membraneinheit (4, 5, 6), über den ein Gasstrom entnommen werden kann, der einen erhöhten Anteil an Methan enthält;
bd) einen Permeatauslass auf der Niedrigdruckseite der Membraneinheit (4, 5, 6), über den ein Gasstrom entnommen werden kann, der einen erniedrigten Anteil an Methan enthält;
c) einer ersten Auslassleitung (3), über welche ein aus hochreinem Methan bestehender Produktgasstrom einer weiteren Verwendung zuführbar ist;
d) einer zweiten Auslassleitung (22), über welche ein nur noch einen geringen Methananteil aufweisender Offgasstrom an die Umgebung abgegeben werden kann;
**dadurch gekennzeichnet, dass**
e) ein erster Qualitätssensor (17) in der ersten Auslassleitung (3) vorgesehen ist, der ein für den Methananteil im Produktgasstrom repräsentatives Signal erzeugt;
f) eine Vakuumpumpe (15) vorgesehen ist, die saugseitig mit der Niedrigdruckseite einer Membraneinheit (5) und druckseitig direkt oder indirekt mit dem Einlass des Verdichters (9) verbunden ist;
g) eine erste Steuereinrichtung (26) vorgesehen ist, welcher das Signal des ersten Qualitätssensors (17) zuführbar ist und deren Ausgangssignal die Saugleistung der Vakuumpumpe (15) so regelt, dass ein vorgegebener Wert für den Methananteil im Produktgas nicht unterschritten wird.

3. Anlage nach Anspruch 2, **dadurch gekennzeichnet,**
**dass** mindestens zwei Membraneinheiten (4, 5) vorgesehen sind,
wobei
der Einlass der ersten Membraneinheit (4) mit dem Auslass des Verdichters (9) verbunden ist;
der Retentatauslass der ersten Membraneinheit (4) mit dem Einlass der zweiten Membraneinheit (5) verbunden ist;
die Vakuumpumpe (15) mit dem Permeatauslass der zweiten Membraneinheit (5) verbunden ist;
der Retentatauslass der zweiten Membraneinheit (5) mit der ersten Auslassleitung (3) für das Produktgas verbunden ist.

4. Anlage nach Anspruch 3, **dadurch gekennzeichnet,**
**dass** der Permeatauslass der ersten Membraneinheit (4) mit dem Einlass einer dritten Membraneinheit (6) verbunden ist, deren Retentatauslass über ein steuerbares Regelventil (20) direkt oder indirekt mit dem Einlass des Verdichters (9) und deren Permeatauslass mit der zweiten Auslassleitung (22) für das Offgas verbunden ist;
wobei
in der zweiten Auslassleitung (22) für das Offgas ein zweiter Qualitätssensor (23) vorgesehen ist, der ein für den Methananteil im Offgas repräsentatives Signal erzeugt;
eine zweite Steuereinrichtung (27) vorgeshen ist, welcher das Signal des zweiten Qualitätssensors (23) zuführbar ist und deren Ausgangssignal das Regelventil (20) so ansteuert, dass ein vorgegebener Wert für den Methananteil im Offgas nicht überschritten wird.
